# EUROPEAN PATENT SPECIFICATION

(11) **EP 1 707 638 B1**
(45) Date of publication and mention of the grant of the patent: **29.11.2017**
(21) Application number: 04807697.0
(22) Date of filing: 24.12.2004
(51) Int. Cl.: C12Q 1/68, G01N 33/48, C12N 15/00

(54) **METHOD OF MULTIPLEX MICROORGANISM DETECTION**
VERFAHREN ZUM NACHWEIS VON MIKROORGANISMEN MITTELS MULTIPLEX-ANALYSE
PROCEDE DE DETECTION MULTIPLEX DE MICRO-ORGANISMES

(30) Priority: 26.12.2003 JP 2003435943
(43) Date of publication of application: 04.10.2006
(73) Proprietor: Prima Meat Packers, Ltd., Tokyo 140-8529 (JP); National Agriculture and Food Research Organization, Tsukuba-shi, Ibaraki 305-8517 (JP)
(72) Inventor: HORIKOSHI, Naoko, c/o Prima Meat Packers, Ltd., Tsuchiura-shi, Ibaraki 300-0841 (JP); KAWASAKI, Susumu, c/o Food Research Institute, NARO, Tsukuba-shi, Ibaraki 305-8642 (JP); OKADA, Yukio, c/o Prima Meat Packers, Ltd., Tsuchiura-shi, Ibaraki 300-0841 (JP); TAKESHITA, Kazuko, c/o Prima Meat Packers, Ltd., Tsuchiura-shi, Ibaraki 300-0841 (JP); SAMESHIMA, Takashi, c/o Prima Meat Packers, Ltd., Tsuchiura-shi, Ibaraki 300-0841 (JP); KAWAMOTO, Shinichi, Food Research Institute, NARO, Tsukuba-shi, Ibaraki 305-8642 (JP); ISSHIKI, Kenji, c/o Food Research Institute, NARO, Tsukuba-shi, Ibaraki 305-8642 (JP)
(74) Representative: Nederlandsch Octrooibureau
(86) International application number: PCT/JP2004/019340
(87) International publication number: WO 2005/064016

(56) References cited:
- JP-A- 2005 034 121
- KR-A- 2002 069 573
- AZNAR R ET AL: "On the Specificity of PCR Detection of Listeria monocytogenes in Food: a Comparison of Published Primers" SYSTEMATIC AND APPLIED MICROBIOLOGY, XX, XX, vol. 25, no. 1, 2002, pages 109-119, XP004957422 ISSN: 0723-2020
- COCOLIN L ET AL: "A MULTIPLEX-PCR METHOD TO DETECT ENTEROHEMORRHAGIC (EHEC) AND ENTEROPATHOGENIC (EPEC) ESCHERICHIA COLI IN ARTIFICIALLY CONTAMINATED FOODS" INTERNATIONAL JOURNAL OF HYGIENE AND ENVIRONMENTAL HEALTH, URBAN U. FISCHER, JENA, DE, vol. 203, no. 2, October 2000 (2000-10), pages 159-164, XP009015102 ISSN: 1438-4639
- TAGURI T. ET AL: 'Multiplex PCR o Mochiita Shokuchudoku Kiin Saikin no Ikkatsu Kenshutu Hoho' ANNUAL REPORT OF NAGASAKI PREFECTURAL INSTITUTE OF PUBLIC HEALTH AND ENVIRONMENTAL SCIENCES vol. 48, 2003, pages 43 - 56, XP002997674
- BRASHER C.W. ET AL: 'Detection of Microbial Pathogens in Shellfish with Multiplex PCR' CURR.MICROBIOL. vol. 37, no. 2, August 1998, pages 101 - 107, XP002277350
- PITCHER D G ET AL: "RAPID EXTRACTION OF BACTERIAL GENOMIC DNA WITH GUANIDIUM THIOCYANATE", LETTERS IN APPLIED MICROBIOLOGY, OXFORD, GB, vol. 8, no. 4, 1 January 1989 (1989-01-01) , pages 151-156, XP009044138, ISSN: 1472-765X

### SEQUENCE LISTING

<110> Prima Meat Packers, Ltd.
<120> Multiple Detecting Method for Microorganism
<130> 2004C2278
<150> JP2003-435943
   <151> 2003-12-26
<160> 23
<170> PatentIn version 3.1
<210> 1
   <211> 20
   <212> DNA
   <213> Artificial
<220>
   <223> primer
<400> 1
   ggcggattag acttcggcta 20
<210> 2
   <211> 20
   <212> DNA
   <213> Artificial
<220>
   <223> primer
<400> 2
   cgttttggca ctatttgccc 20
<210> 3
   <211> 25
   <212> DNA
   <213> Artificial
<220>
   <223> primer
<400> 3
   gggagtccag gttgacggaa aattt 25
<210> 4
   <211> 26
   <212> DNA
   <213> Artificial
<220>
   <223> primer
<400> 4
   gtcacggaag aagagaaatc cgtacg 26
<210> 5
   <211> 20
   <212> DNA
   <213> Artificial
<220>
   <223> primer
<400> 5
   cggaggttcc gcaaaagatg 20
<210> 6
   <211> 20
   <212> DNA
   <213> Artificial
<220>
   <223> primer
<400> 6
   cctccagagt gatcgatgtt 20
<210> 7
   <211> 21
   <212> DNA
   <213> Artificial
<220>
   <223> primer
<400> 7
   atcattgacg attgtagcac c 21
<210> 8
   <211> 21
   <212> DNA
   <213> Artificial
<220>
   <223> primer
<400> 8
   acatgaggag cattaacttc g 21
<210> 9
   <211> 20
   <212> DNA
   <213> Artificial
<220>
   <223> primer
<400> 9
   gggtcgttct acattgacag 20
<210> 10
   <211> 20
   <212> DNA
   <213> Artificial
<220>
   <223> primer
<400> 10
   ttccctttcc agtacgcttc 20
<210> 11
   <211> 20
   <212> DNA
   <213> Artificial
<220>
   <223> primer
<400> 11
   gtatttggag acatgggagc 20
<210> 12
   <211> 21
   <212> DNA
   <213> Artificial
<220>
   <223> primer
<400> 12
   actaatgaca cgattcgttc c 21
<210> 13
   <211> 19
   <212> DNA
   <213> Artificial
<220>
   <223> primer
<400> 13
   cggacagtag ttataccac 19
<210> 14
   <211> 19
   <212> DNA
   <213> Artificial
<220>
   <223> primer
<400> 14
   ctgctgtcac agtgacaaa 19
<210> 15
   <211> 21
   <212> DNA
   <213> Artificial
<220>
   <223> primer
<400> 15
   agctttggtc gtaaaataag g 21
<210> 16
   <211> 20
   <212> DNA
   <213> Artificial
<220>
   <223> primer
<400> 16
   gatgcccaaa gcagagagat 20
<210> 17
   <211> 21
   <212> DNA
   <213> Artificial
<220>
   <223> primer
<400> 17
   caaactgcta acacagctac t 21
<210> 18
   <211> 21
   <212> DNA
   <213> Artificial
<220>
   <223> primer
<400> 18
   gcacttgaat tgctgttatt g 21
<210> 19
   <211> 19
   <212> DNA
   <213> Artificial
<220>
   <223> primer
<400> 19
   accaatggga tccacaaga 19
<210> 20
   <211> 19
   <212> DNA
   <213> Artificial
<220>
   <223> primer
<400> 20
   gagctgagct atgtgcgat 19
<210> 21
   <211> 23
   <212> DNA
   <213> Artificial
<220>
   <223> probe
<400> 21
   cggatgattt gtggcacgag aaa 23
<210> 22
   <211> 26
   <212> DNA
   <213> Artificial
<220>
   <223> probe
<400> 22
   tctggcatta tcgatcagta ccagcc 26
<210> 23
   <211> 28
   <212> DNA
   <213> Artificial
<220>
   <223> probe
<400> 23
   agttcaaatc atcgacggca acctcgga 28

## Description

### Technical Field

The present invention relates to a method for multiplex microorganism detection. This method can detect microorganisms including pathogenic E. coli 0157, Listeria monocytogenes and Salmonella spp. existing in foods, with high sensitivity comparable or even superior to official methods (KO̅TEI-HO̅:Japan), comprising the steps of amplifying a plurality of target genes in a single PCR reaction tube and analyzing the same.

### Background Art

Conventionally, methods for multiplex microorganism detection using multiplex PCR are well known. For example the following methods have been reported: a method for multiplex detection of bacteria of pathogenic Escherichia coli 0157, Salmonella spp. and Listeria monocytogenes, targeting vegetables and fruits (for example, see non-patent document 1); a method for multiplex detection of pathogenic Escherichia coli 0157 and Salmonella spp. in foods (for example, see non-patent document 2); a method for multiplex detection of various bacteria of pathogenic Escherichia coli 0157, Salmonella spp., Listeria monocytogenes and Campylobacter spp., targeting milk (for example, see non-patent document 3); a method for multiplex detection of Salmonella spp. and Listeria monocytogenes in foods (for example, see non-patent document 4); a method for multiplex detection of Escherichia coli including pathogenic Escherichia coli 0157, in foods (for example, see non-patent document 5); and a method for multiplex detection of bacteria of pathogenic Escherichia coli 0157, Salmonella spp. and Listeria monocytogenes, targeting milk (for example, see non-patent document 6). Further, primers for detecting Escherichia coli (for example, see patent document 1) and primers for detecting O-antigen of pathogenic Escherichia coli 0157 (for example, see patent document 2) are known as primers for multiplex PCR.

Furthermore, the following methods for extracting DNAs of microorganisms are known: a method using lytic enzyme Achromopeptidase and the like for mycobacterium such as tubercle bacilli (for example, see patent document 3); a method using lytic enzyme Achromopeptidase and the like for Gram negative bacteria and Gram positive bacteria (for example, see patent document 4); a method using proteinase K or Achromopeptidase and the like for Legionella (for example, see patent document 5); and a method using protein denaturant, reducing agent, surfactant, chelator and the like for Escherichia coli and the like (for example, see patent document 6).

Patent document 1: Japanese Laid-Open Patent Application No. 2001-95576
Patent document 2: Japanese Laid-Open Patent Application No. 11-332599
Patent document 3: Japanese Laid-Open Patent Application No. 6-165676
Patent document 4: Japanese Laid-Open Patent Application No. 9-500793
Patent document 5: Japanese Laid-Open Patent Application No. 5-317033
Patent document 6: Japanese Laid-Open Patent Application No. 6-289016
Non-patent document 1: Japanese Journal of Food Microbiology, Vol. 19, No. 2, 47-55, 2002
Non-patent document 2: Journal of Industrial Microbiology & Biotechnology, 21, 92-98, 1998
Non-patent document 3: Milchwissenschaft, 55(9), 500-503, 2000
Non-patent document 4: Journal of Food Protection, Vol. 64, No. 11, 1744-1750, 2001
Non-patent document 5: Molecular and Cellular Probes, 13, 291-302, 1999
Non-patent document 6: Food Microbiology, 20, 345-350, 2003

### Disclosure of the Invention

### Object to be solved by the present invention

Methods for detecting one type of microorganism by PCR have been already established, while methods for detecting simultaneously multiple microorganisms are being considered in the food sector but no methods sufficiently reliable have been established so far. The object of the present invention is to provide a method for multiplex microorganism detection that can detect contaminating microorganisms including pathogenic Escherichia coli 0157, Listeria monocytogenes and Salmonella spp. existing in foods, with high sensitivity comparable, or even superior to official methods, comprising the steps of amplifying a plurality of target genes with a single PCR reaction tube, and analyzing the same. In other words, the invention provides a method for multiplex microorganism detection that can detect contaminating microorganisms including pathogenic Escherichia coli, Listeria monocytogenes and Salmonella spp., by using multiplex PCR, known as a PCR method using plural pairs of primers in combination, easily, with high sensitivity and good reproducibility

### Means to solve the object

### (Culture)

Using official methods, harmful pathogenic bacteria are not detectable in food ("negative") in foods (when not present in 25 g of food. Therefore a comparable or even superior accuracy compared to the one of official methods is required for the detection of these bacteria. Enrichment culture is essential to detect contaminating microorganisms that are present in minute amounts such as 1 CFU level in 25 g of food. When performing enrichment culture, usually, culture is conducted by using a selective medium for each target bacterium. However, in order to detect multiple microorganisms simultaneously, considerations were made to proliferate simultaneously multiple microorganisms in one single type of medium, even for enrichment culture. It is preferable to determine culture conditions leading to detection in the shortest possible time (for example, 24 hours or less), and therefore, the selection of medium becomes particularly important. It is relatively easy to proliferate bacteria simultaneously, when the target microorganisms belong to the same family or bacterial species of same genus, or if their growing properties are alike, but it is difficult for microorganisms of diverse types and having different growing properties. For example, when pathogenic Escherichia coli 0157, Salmonella spp. and Listeria monocytogenes are the targets to be detected, a discrepancy was found among these three pathogenic bacteria, Listeria grows at a low temperature and proliferates slower compared to Salmonella spp. and 0157.

Therefore, medium having preferable nutrient source for Listeria, with low carbohydrate and high buffer ability were considered so that especially Listeria can proliferate sufficiently even when other bacteria are present. As a result, Listeria proliferated the fastest of the three contaminating bacteria, in medium No. 17 (tryptose 10 g, meat extract 5 g, yeast extract 5 g, sodium chloride 5 g, glucose 0.5 g, disodium phosphate 7 g, monopotassium phosphate 15 g/1 L), compared to Trypto-soya broth or BHI (Brain Heart Infusion), or BPW (Buffered peptone water), wherein Salmonella spp. and 0157 proliferated faster than Listeria. This result suggested that medium No. 17 was preferable for detecting simultaneously these 3 types of bacteria.

(DNA) In order to perform a PCR reaction, DNAs are extracted, and a lytic operation is necessary to that end. Lysis of Gram positive bacteria is significantly more difficult compared to the lysis of Gram negative bacteria. This is due to a difference in the main cell wall component: the main cell wall component of Gram positive bacteria is thicker and has a higher-density peptide glycan layer compared the cell wall of Gram negative bacteria. The present technique was difficult as Listeria, a Gram positive bacteria, was to be detected simultaneously in addition to a Gram negative bacteria including Salmonella spp. and 0157. In addition, the microorganisms were extracted from various food products. Therefore, it seems very difficult to determine one single efficient lysis method. Particularly, in samples as represented by meat of livestock, which have a high protein and fat contents, and have individual differences, lysis is not expected to be performed at a constant efficiency, and therefore, DNA extraction was expected to be difficult to realize. Further consideration revealed that some Listeria, cells were not disrupted (=DNAs could not be extracted) using only lysozyme as lytic enzyme, depending on the culture medium, such as Trypto-soya broth or Mueller Hinton Broth. It is estimated that culture conditions would have to be adjusted depending on the type or the deterioration level of foods, even when using the same medium. Further, in some cases, as it is necessary to use media with better recovering ability, extraction methods wherein cells can be disrupted under any circumstances were necessary.

Boiling and alkaline-SDS methods are known as general DNA extraction methods. However, it was confirmed that Listeria could not be extracted with high sensitivity using the boiling method. SDS is known as a surfactant that is easy to use in DNA extraction. However, it is a strong PCR inhibitor. Therefore it is necessary to remove it completely after having been used for extraction. For obtaining similar effects and results from an unskilled experimenter as from a skilled artisan, substances that affect the reaction even by a contamination at a low concentration, such as SDS, are not preferable.SDS has been shown to be a factor that can be the cause of a variability in the extraction efficiency when extraction is performed by an unskilled versus a skilled person. In addition, phenol and chloroform are organic solvents dangerous and harmful to human body. But their use allows a better purification level of DNA. However, it can be easily estimated that there will be technical individual differences in extraction efficiency, and thus a constant sensitivity cannot be assured. Furthermore, as a particular waste liquid treatment is necessary, it can be said that it is not an extraction method suitable for examination in food manufacturing sites. Therefore, it was necessary to develop a method that is easy and safe so that it can be performed in food manufacturing sites, enabling DNA extraction even from high-protein containing foods as represented by meat of livestock, that can be easily operated by unskilled or skilled artisans, and that gives a constant DNA extraction efficiency (i.e. detection sensitivity).

It was shown that bacteria could be lysed completely, by removing large food scraps by passing the culture solution through a 5 µm-filter, adding a lytic enzyme solution (mixed solution of Achromopeptidase and lysozyme), treating the mixture at 37°C for 1 h, and adding a mixed solution of surfactant (Tween 20) and protein denaturant (Guanidine isothiocyanate). Moreover, it was shown that bacteria could also be completely lysed by using a bacteriocin having lytic activity such as Enterolysine instead of Achromopeptidase. DNAs were extracted by removing insoluble fractions by centrifugation, and performing alcohol precipitation. It was shown that the following treatment order was extremely preferable: performing lysozyme treatment preferably prior to or simultaneously with Achromopeptidase; performing Enterolysine treatment preferably prior to or simultaneously with lysozyme treatment; subsequently performing Tween 20 treatment followed by Guanidine isothiocyanate treatment, or performing Tween 20 treatment and Guanidine isothiocyanate treatment simultaneously. It was further shown that as Tween 20 has a high viscosity, it was difficult to add it separately, and thus adding it as a mixture was preferable.

Further, even if a phenol or chloroform treatment is not performed, protein being soluble to a level sufficient to be detected by PCR without problem can be removed by alcohol precipitation or according to the added level of DNA extraction solution (2 µl per 50 µl of PCR reaction solution). As Tween 20 is also used in PCR reaction solution, Tween 20 is preferably used instead of SDS. In addition, Tween 20 is easy to be used by unskilled experimenters. Moreover, these extraction methods can be applied for extraction of each bacterium alone, independently. Furthermore, the following methods have been tried: (1) Achromopeptidase alone; (2) lysozyme alone; (3) Enterolysine alone; (4) Achromopeptidase treatment followed by treatment with guanidine isothiocyanate + Tween 20; (5) lysozyme treatment followed by guanidine isothiocyanate + Tween 20; (6) Enterolysine treatment followed by treatment with guanidine isothiocyanate + Tween 20; (7) proteinase K, (8) proteinase K treatment followed by treatment with guanidine isothiocyanate + Tween 20; (9) guanidine isothiocyanate + Tween 20 treatment; (10) guanidine isothiocyanate + Tween 20 + heating treatment. The best extraction results
(sensitivity)for Listeria were obtained with the method comprising treatment with Achromopeptidase and lysozyme in combination, or treatment with Enterolysine and lysozyme in combination, followed by treatment with Tween 20 and guanidine isothiocyanate.

(PCR reaction) Multiplex PCR was applied as a method for detecting simultaneously multiple bacteria. For multiplex PCR method which is a PCR method performed by combining several primers, pairing primers were selected and used that do not mutually produce primer dimer, or that do not produce identification bands that will interfere or overlap each other, and that have similar melting temperature. It has been revealed that difference arises to the ease of reaction or detection limit according to the primer selection or mixing ratio. When performing multiplex PCR, it is necessary to adjust the primer mixing ratio in order to detect bands of 3 types of bacteria to be used in later determination, with a similar intensity in the electrophoresis image. When the 3 types of bacteria have the same DNA concentration (20 pg), it was adjusted so that the bands of 3 types of bacteria could be detected with a similar intensity. For example, when DNA consisting of base sequences shown by SEQ ID Nos: 1 to 6 is used as a primer, the most preferable mixing ratio of 6 primers was as follows: 120 nM each of Salmonella spp. primers, 100 nM each of Listeria primers; 80 nM each of 0157 primers. Further, when DNA consisting of base sequences shown by SEQ ID Nos: 5 to 10 is used as a primer, the most preferable mixing ratio of 6 primers was: 60 nM each of Salmonella spp. primers; 60 nM each of Listeria primers; 240 nM each of 0157 primers. Moreover, a mixing ratio with lower concentration, i.e. 30 nM each of Salmonella spp. primers, 25 nM each of Listeria primers and 20 nM each of O157 primers (when DNA consisting of base sequences shown by SEQ ID Nos: 1 to 6 is used); and 15 nM each of Salmonella spp. primers, 15 nM each of Listeria primers and 60 nM each of 0157 primers (when DNA consisting of base sequences shown by SEQ ID Nos: 5 to 10 is used) were also investigated. However, as visual detection by electrophoresis was difficult though possible, the above first mentioned preferred concentrations were found to be preferable.

Further, it is known that target genes can be amplified to approximately 10⁻⁸ M as final production level by PCR reaction. Normally, approximately 200 nM of primer is added to 1 bacterium in PCR reaction, while in case of multiple detection, it was revealed that this primer level is obviously excessive to be added to each bacterium. Particularly, in case of multiplex reaction, it is highly possible that only results of dominant reaction caused by generated products due to the excessive primer (including non-targeted product) are obtained. Thus, the relationship between the control of the final product level with the multiplex reaction was considered by controlling the primer level in PCR reaction. Naturally, as non-amplified products as represented by primer dimer also consume primers in PCR reaction, it was revealed that although there is a lower limit of the concentration, more multiplex reactions could be achieved successively by determining the primer concentration to the minimum sensitivity of the detecting machine. In other words, by adjusting the lower limit primer concentration to the sensitivity of the detecting machine, detection of more microorganisms can be expected. By considering the detection machine limit for electrophoresis, fluorescent probe method, or capillary electrophoresis method, the reaction was to be conducted at approximately 50 nM or more, and detection of 3 types of pathogenic bacteria was confirmed. It is estimated that by increasing the detection machine sensitivity, this concentration can be set to a lower level, and thus, more targets can be detected at once. It was also revealed that if a more sensitive method for detecting amplification products is realized, by controlling the final product level in view of the above, simultaneous multiple detection by more Multiplex PCR can be realized.

In other words, the present invention relates to: (1) a method for multiplex microorganism detection which is a method for detecting two or more microorganisms having different properties in foods, with high sensitivity comparable or even superior to official methods, by amplifying a plurality of target genes in a single PCR reaction tube and analyzing the same, comprising the following steps:
(A) a step for extracting DNA of the target microorganisms to be detected, by treating with at least a lytic enzyme and /or a bacteriocin having a lytic activity, a surfactant and a protein-denaturing agent; and
(B) a step for carrying out Multiplex PCR after having mixed primers specific to the target microorganisms to be detected.
According to another preferred embodiment defined as (2), the method for multiplex microorganism detection as defined above as (1) comprises an additional step: an additional step is performed for culturing microorganisms under such culture conditions as allowing the proliferation of 1 CFU/100 g microorganisms before the culture to achieve a level of 10³ CFU/ml or more after culturing for 24 h, said additional step being performed prior to step (A) (the step of extracting DNA of the target microorganisms to be detected).
According to another preferred embodiment defined as (3), the method for multiplex microorganism detection as defined in (1) or (2),is such that the two or more microorganisms with different properties comprise Listeria monocytogenes. Optionally, and according to a more preferred embodiment defined as (4) the method for multiplex microorganism detection as defined in(3) is performed using the specific primers consisting of base sequences shown by SEQ ID Nos: 5 and 6.
According to another preferred embodiment defined as (5), the method for multiplex microorganism detection as defined in (1) or (2), is such that the two or more microorganisms with different properties comprise pathogenic Escherichia coli 0157.Optionally, and according to a more preferred embodiment defined as (6), the method for multiplex microorganism detection as defined in(5), is performed using the specific primers consisting of base sequences shown by SEQ ID Nos: 1 and 2, or SEQ ID Nos: 7 and 8.
According to another preferred embodiment defined as (7), the method for multiplex microorganism detection as defined in(1) or (2) is such that the two or more microorganisms with different properties comprise Salmonella spp.. Optionally, and according to a more preferred embodiment defined as (8) the method for multiplex microorganism detection as defined in(7) is performed using the specific primers consisting of base sequences shown by SEQ ID Nos: 3 and 4, or SEQ ID Nos: 9 and 10.

Moreover, the present invention relates to another preferred embodiment defined as (9), wherein the method for multiplex microorganism detection is according to any one of previously defined preferred methods as (1) to (8), and wherein the microorganisms are cultured in such culture conditions allowing the pH to reach a value of 5.1 or higher after the completion of the culture.
According to another preferred embodiment defined as (10), wherein the method for multiplex microorganism detection is according to any one of previously defined preferred methods as (1) to (9), and wherein the microorganisms are cultured in a medium with a glucose concentration of 0.15% or less, and/or in a medium with a concentration of phosphate-buffer solution of 50 mM or more or in a medium with a buffer ability, which is similar to the one of a medium having a concentration of phosphate-buffer solution of 50 mM or more.
According to another preferred embodiment defined as (11), wherein the method for multiplex microorganism detection is according to any one of previously defined preferred methods as (1) to (10), and wherein the extraction step (A) is performed by treating with a lytic enzyme and/or a bacteriocin having a lytic activity, further treating with a surfactant and a protein-denaturing agent, removing insoluble fractions by centrifugation, and depositing DNA by alcohol precipitation.
According to another preferred embodiment defined as (12) wherein the method for multiplex microorganism detection is according to any one of previously defined preferred methods as (1) to (11), and wherein the lytic enzyme is Achromopeptidase and/or lysozyme.
According to another preferred embodiment defined as (13) wherein the method for multiplex microorganism detection is according to any one of previously defined preferred methods as (1) to (12), and wherein the bacteriocin having a lytic activity is Enterolysine.
According to another preferred embodiment defined as (14) wherein the method for multiplex microorganism detection is according to any one of previously defined preferred methods as (1) to (13), and wherein the surfactant is ethyleneoxide condensate of sorbitan monolaurate.
According to another preferred embodiment defined as (15)wherein the method for multiplex microorganism detection is according to any one of previously defined preferred methods as (1) to (14),and wherein the protein-denaturing agent is Guanidine isothiocyanate.
According to another preferred embodiment defined as (16) wherein the method for multiplex microorganism detection is according to any one of previously defined preferred methods as (1) to (15), and wherein Multiplex PCR is performed by combining DNA consisting of base sequences shown by SEQ ID NOs: 1 to 6 at a total concentration of 750 nM or less as a primer.
According to another preferred embodiment defined as (17) wherein the method for multiplex microorganism detection is according to any one of previously defined preferred methods as (1) to (15), and wherein Multiplex PCR is performed by combining DNA consisting of base sequences shown by SEQ ID NOs: 5 to 10 at a total concentration of 750 nM or less as a primer.
According to another preferred embodiment defined as (18) wherein the method for multiplex microorganism detection is according to any one of previously defined preferred methods as (1) to (17),and wherein the food is edible meat or processed meat product.

### Effect of the present invention

According to the present invention, microorganisms existing in foods, including pathogenic Escherichia coli 0157, Listeria monocytogenes and Salmonella spp., can be easily detected with high sensitivity comparable or even superior to official methods, by amplifying a plurality of target genes in a single PCR reaction tube and analyzing the same.

### Best Mode of Carrying Out the Invention

The method for multiplex microorganism detection of the present invention is not particularly limited as long as it is a method for detecting two or more microorganisms with different properties in food including edible meat, processed meat products, milk and vegetables, at a sensitivity comparable or even superior to official methods, by amplifying a plurality of target genes in a single PCR reaction tube and analyzing the same, comprising the following steps: (A) a step for extracting DNA of the target microorganisms to be detected, by treating with at least a lytic enzyme and/or a bacteriocin having a lytic activity, further treating with a surfactant and a protein-denaturing agent denaturant; and (B) a step for carrying out Multiplex PCR after having mixed primers specific to the target microorganisms to be detected. Preferably, an additional step for culturing microorganisms is performed, wherein the microorganisms are cultured under such culture conditions as allowing the proliferation of 1 CFU/100 g microorganisms before the culture to achieve a level of 10³ CFU/ml or more after culturing for 24 h. This additional step is performed prior to the above step (A)for extracting DNA of the target microorganisms to be detected. Further, target microorganisms to be detected are not limited as long as they are contaminating microorganisms in foods, and specifically include, Listeria monocytogenes, pathogenic Escherichia coli 0157, Salmonella spp., Campylobacter spp., Vibrio parahaemolyticus, Staphylococcus aureus, Yersinia, Coliform bacteria, Bacillus cereus, Vibrio cholerae, Shigella, Clostridium botulinum.In addition, official methods for detecting microorganisms as described in "Guidance for food hygiene inspection" (Japan Food Hygiene Association, 1990), are exemplified in Example 9.

According to the multiplex microorganism detection method of the present invention, it is possible to detect microorganisms that have been contaminated in minute amounts such as 1 CFU level in 25 g of food. In addition in the multiplex microorganism detection method of the present invention, the culturing process of the contaminating microorganisms in food is very important. The culture conditions leading to an enrichment of contaminating microorganisms in food are exemplified as follows: the culture conditions are such as allowing the proliferation of 1 CFU/100 g microorganism before the culture to achieve a level of 10³ CFU/ml or more microorganisms after culturing for 48 h, preferably after culturing for 30 h, more preferably after culturing for 24 h, and most preferably after culturing for 18 h. It is further preferable to culture the microorganisms in such culture conditions allowing the pH to reach a value of 5.1 or higher after the completion of the culture. The culturing step can be carried out using a medium having buffer ability, or a medium with a glucose concentration of 0.15% or less, and/or a medium with a concentration of phosphate-buffer solution of 50 mM or more or in a medium with a buffer ability,which is similar to the one of a medium having a concentration of phosphate-buffer solution of 50 mM or more. Other buffer solutions than phosphate buffer solution may be used such as citrate buffer solution, acetate buffer solution, lactate buffer solution, tartrate buffer solution, malate buffer solution, tris buffer solution, MOPS buffer solution and MES buffer solution.

In the multiplex detection method of the present invention, the process for extracting DNA of contaminating microorganisms in foods is preferably performed on microorganisms which have been cultured and proliferated. The DNA extraction process is not particularly limited as long as it is a process for extracting DNA of target microorganisms to be detected, by treating with at least a lytic enzyme and/or a bacteriocin with a lytic activity, further treating with a surfactant and a protein-denaturing agent. Subsequently, the DNA is preferably extracted by alcohol precipitation and insoluble fractions are preferably removed by centrifugation. Examples of the above mentioned lytic enzymes include: Achromopeptidase, lysozyme, proteinase K, chitosanase, chitinase, -1,3-Glucanase, Zymolyase and Cellulase. Examples of bacteriocin having lytic activity include Enterolysine and helveticine. One or more of these can be used, while Achromopeptidase, lysozyme, Enterolysine, or a combination thereof, for example simultaneous use of Achromopeptidase and lysozyme, simultaneous use of Enterolysine and lysozyme, can be preferably exemplified. As surfactants, anion surfactant, cation surfactant, amphoteric surfactant, nonionic surfactant can be used. Among these, ethylene oxide condensate of sorbitan monolaurate which is a nonionic surfactant, more specifically Tween 20,are preferably used. As protein-denaturing agent, guanidine isothiocyanate, urea, guanidine hydrochloride, trichloroacetate, SDS, Triton X-100 and deoxycholate can be used. One or more of these protein-denaturing agent can be used, while guanidine isothiocyanate is preferably used since it gives good results in bacteriolysis and it is easy to handle. Extract/deposit of DNA from lysates can be performed by commonly known methods such as removing insoluble fractions by centrifugation, and then performing alcohol precipitation.

In the multiplex detection method of the present invention, a process is provided which comprises the steps of mixing the above extracted DNA and primers specific to target microorganisms to be detected, to perform multiplex PCR. As for primers used, it is preferable to select primers specific to target microorganisms to be detected, which is a pairing primers having similar melting temperature that do not mutually produce primer dimer, or wherein their identification bands do not interfere or overlap each other. Further, it is preferable to adjust the mixing ratio of the primers so that bands appearing in electrophoresis used for the subsequent determination, can be detected with similar intensity. Examples of primer sets specific for pathogenic Escherichia coli O157 are DNA consisting of base sequences shown by SEQ ID NOs: 1 and 2, SEQ ID NOs: 7 and 8, SEQ ID NOs: 11 and 12, SEQ ID NOs: 13 and 14, etc.. Examples of primer sets specific for Salmonella spp. are DNA consisting of base sequences shown by SEQ ID NOs: 3 and 4, SEQ ID NOs: 9 and 10, SEQ ID NOs: 15 and 16, etc.. Examples of primer sets specific for Listeria monocytogenes are DNA consisting of base sequences shown by SEQ ID NOs: 5 and 6, SEQ ID NOs: 17 and 18, SEQ ID NOs: 19 and 20, etc.. All these exemplified primer sets can be used in combinations. Among these combinations, the combination of DNA consisting of base sequences shown by SEQ ID NOs: 1 to 6, or SEQ ID NOs: 5 to 10 is the most preferable, and in this case, it is preferable to mix primers at a total concentration of 750 nM or less. When SEQ ID NOs: 1 to 6 are used, the preferable mixing ratio of the 6 primers is: 120 nM each of Salmonella spp. primers, 100 nM each of Listeria primers, 80 nM each of 0157 primers. When SEQ ID NOs: 5 to 10 are used, the preferable mixing ratio is: 60 nM each of Salmonella spp. primers, 60 nM each of Listeria primes, and 240 nM each of 0157 primers.

The detection method used following PCR can be for example, electrophoresis, fluorescent probe method, capillary electrophoresis method, or quantitative PCR method. Particularly, in case of quantitative PCR, it can be detected by using DNA consisting of base sequences shown by SEQ ID NOs: 5 to 10 as primer, and by using DNA consisting of base sequences shown by SEQ ID NOs: 21 to 23 as fluorescent probe.

### Example 1

### (Simultaneous culture in an existing medium)

Escherichia coli O157:H7 ATCC43894, Salmonella spp. enteritidis IFO3313 and Listeria monocytogenes ATCC49594 were used for pathogenic Escherichia coli, Salmonella spp. and Listeria monocytogenes, respectively. Further, as meat-derived bacteria, 4 strains, Pseudomonas fragi, Citrobacter freundii, Lactobacillus viridescens and Leuconostoc mesenteroides were used. Two media were used as test medium:Trypto-soya broth (TSB; Nissui Pharmaceutical) and Buffered Peptone Water (BPW; peptone 10 g, sodium chloride 5 g, sodium hydrogenphosphate 3.5 g, potassium dihydrogenphospate 1.5 g/1 L).

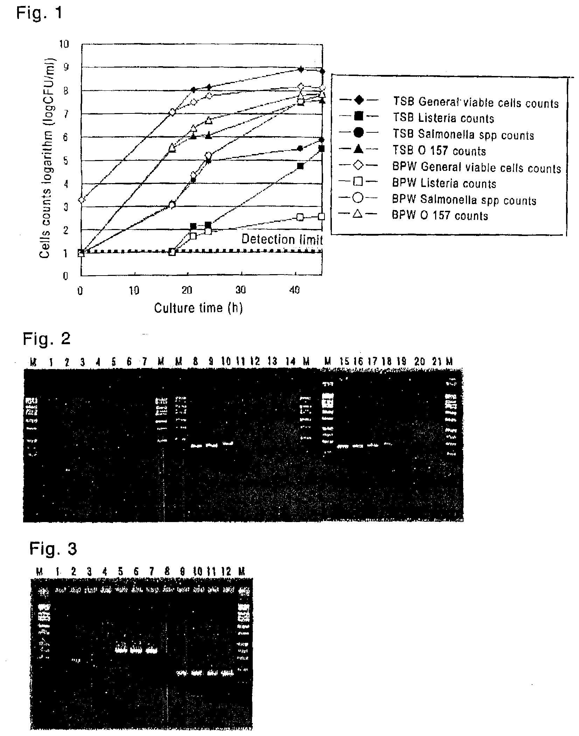

Meat-derived bacteria, and 1 CFU/100 ml of each of the pathogenic Escherichia coli 0157, Salmonella spp., and Listeria monocytogenes were inoculated in the test medium till each bacterium reaches 10⁴ CFU/100 ml. After culturing at 35°C, viable cells of 0157, Salmonella spp. and Listeria were counted chronologically. For estimating general viable cell counts, the total colony counts were measured after culturing at 35°C for 48 h by using a standard agar medium (Nissui Pharmaceutical. For estimating 0157 counts, the Escherichia coli-like colony counts were measured after culturing at 35°C for 24 h by using desoxycholate agar medium (Nissui Pharmaceutical). For estimating Salmonella spp. counts, the Salmonella spp.-like colony counts were measured after culturing at 35°C for 24 h by using MLCB agar medium (Nissui Pharmaceutical). For estimating Listeria counts, the Listeria-like colony counts were measured after culturing at 35°C for 48 h by using LCAM agar medium (Merck). The results are shown in Fig. 1. The results indicate that, particularly the proliferation of Listeria monocytogenes was weak in any of the media tested. By measuring the pH of the culture solution, a decrease of the pH was confirmed.

### Example 2

### (Influence of buffer ability and sugar concentration of the medium)

As it was thought that the weak proliferation of Lysteria monocytogenes in Example 1 was due to the decrease of the pH of the medium during culture, the influence of buffer ability and sugar concentration of the medium on the proliferation of each bacterium was investigated. Disodium phosphate and monopotassium phosphate were added to the base medium (tryptose 10 g, meat extract 5 g, yeast extract 5 g, sodium chloride 5 g/1 L) to adjust the phosphate concentration from 15 mM to 200 mM (pH 6.3), and glucose was added by changing the concentration from 0% to 0.25% to prepare the test medium. Meat-derived bacteria and 1 CFU/100 ml of each of pathogenic Escherichia coli 0157, Salmonella spp. and Listeria moncytegenes, used in Example 1, were inoculated in each test medium till each of these reaches 10⁴ CFU/ml. After culturing at 35°C, counts of general viable cells, 0157, Salmonella spp. and Listeria, as well as the pH levels were measured at several time points (18, 24, 30 and 48 h later). The results are shown in Table 1

As a result, by using either a medium with a glucose concentration of 0.15% or less, or a medium with a phosphate concentration of 50 mM or more, or a medium maintaining the pH after culture at 5.1 or more, all of pathogenic Escherichia coli 0157, Salmonella spp. and Listeria monocytogenes proliferated to 10³ CFU/ml or more (cell counts necessary for detection by PCR) for culturing during at least 18 h or more. For the tests presented thereafter, medium No. 17 of Table 1 (tryptose 10 g, meat extract 5 g, yeast extract 5 g, sodium chloride 5 g, glucose 0.5 g, disodium phosphate 7 g, monopotassium phosphate 15 g/1 L) was selected. As for medium components, nitrogen sources other than tryptose, meat extract or yeast extract, carbon sources other than glucose, substances with buffer ability other than phosphate are also effective according to the existing environment or damage level of the bacteria to be detected. Further, it was more preferable to add inorganic salts, pyruvic acid or pyruvate salt, or surfactants such as Tween as substances promoting proliferation.

**[Table 1]**

| No. | Phosphate concentration (mM) | Glucose concentration (%) | General viable cell counts (CFU/ml) | O157 counts (CFU/ml) | Salmonella spp. counts (CFU/ml) | Listeria counts (CFU/ml) | pH | application |
|---|---|---|---|---|---|---|---|---|
| 1 | 15 | 0 | 2.4×10⁸ | 1.4×10⁷ | 6.2×10⁶ | 2.0×10⁶ | 5.99 | OK |
| 2 | | 0.5 | 1.6×10⁸ | 1.1×10⁷ | 5.6×10⁶ | 1.5×10⁶ | 5.97 | OK |
| 3 | | 1.0 | 3.0×10⁸ | 5.7×10⁶ | 4.3×10⁶ | 4.7×10⁶ | 5.62 | OK |
| 4 | | 1.5 | 2.8×10⁸ | 1.4×10⁵ | 3.0×10⁵ | 3.0×10⁴ | 5.11 | OK |
| 5 | | 2.0 | 3.2×10⁸ | 5.1×10⁴ | 9.1×10² | 6.1×10² | 4.79 | NG |
| 6 | | 2.5 | 3.9×10⁸ | 2.3×10⁴ | 1.2×10³ | 10 | 4.58 | NG |
| 7 | 30 | 0 | 2.1×10⁸ | 3.2×10⁷ | 5.1×10⁶ | 6.2×10⁶ | 6.13 | OK |
| 8 | | 0.5 | 1.8×10⁸ | 2.7×10⁷ | 4.9×10⁶ | 5.9×10⁶ | 6.10 | OK |
| 9 | | 1.0 | 4.4×10⁸ | 2.0×10⁷ | 4.8×10⁶ | 3.0×10⁶ | 5.94 | OK |
| 10 | | 1.5 | 3.1×10⁸ | 4.3×10⁶ | 7.1×10⁶ | 1.6×10⁶ | 5.71 | OK |
| 11 | | 2.0 | 6.8×10⁸ | 4.8×10⁵ | 2.4×10⁴ | 6.9×10³ | 5.40 | OK |
| 12 | | 2.5 | 5.7×10⁸ | 4.7×10⁵ | 1.6×10⁴ | 3.0×10² | 5.03 | NG |
| 13 | 50 | 0.5 | 1.1×10⁸ | 2.4×10⁷ | 5.2×10⁶ | 6.1×10⁶ | 6.12 | OK |
| 14 | | 2.5 | 3.9×10⁸ | 1.1×10⁷ | 5.0×10⁴ | 3.4×10⁴ | 5.62 | OK |
| 15 | 100 | 0.5 | 3.2×10⁸ | 2.5×10⁷ | 5.1×10⁵ | 5.2×10⁶ | 6.10 | OK |
| 16 | | 2.5 | 6.2×10⁸ | 2.0×10⁷ | 4.2×10⁶ | 4.5×10⁶ | 6.02 | OK |
| 17 | 150 | 0.5 | 4.4×10⁸ | 4.1×10⁷ | 5.4×10⁶ | 5.8×10⁶ | 6.17 | OK |
| 18 | | 2.5 | 9.5×10⁷ | 3.2×10⁷ | 5.1×10⁶ | 8.0×10⁶ | 6.22 | OK |
| 19 | 200 | 0.5 | 1.2×10⁸ | 3.2×10⁷ | 5.1×10⁶ | 5.8×10⁶ | 6.17 | OK |
| 20 | | 2.5 | 8.4×10⁷ | 3.8×10⁷ | 6.8×10⁶ | 9.0×10⁶ | 6.15 | OK |

### Example 3

### (DNA extraction method 1)

Each bacteria of pathogenic Escherichia coli 0157, Salmonella spp. and Listeria monocytogenes were inoculated into 10 ml of medium No. 17, and cultured at 35°C for 18 h. Each culture solution was taken to a 1 ml-tube, respectively, centrifuged at 15,000 r.p.m for 5 min to collect bacteria. Lytic enzyme solution {mixed solution of 10 µl of 20 mg/ml Achromopeptidase and 10 µl of 20 mg/ml Lysozyme, and 180 µl of TE buffer [10 mM Tris (hydroxymethyl) aminomethane-hydrochloric acid buffer solution containing 1 mM EDTA, pH 8]} was added to the tube and the tubes were incubated at 37°C for 1 h. Subesequently, 300 µl of lysing agent (4 M guanidine isothiocyanate solution supplemented with 1 to 2% of Tween 20) was added to each tube to dissolve bacteria completely. By observing this solution with a light microscope, it was confirmed that lysis was sufficiently performed. The solution was centrifuged at 15,000 r.p.m for 5 min and 400 µl of the supernatant was transferred to another tube. After precipitating DNA in the lysate with isopropanol, the resultant was centrifuged, and therefore, the intended DNA was obtained. Further, by using Enterolysine instead of Achromopeptidase, it was confirmed that lysis was similarly sufficiently performed.

### Example 4

### (DNA extraction method 2)

Similarly, each bacteria of pathogenic Escherichia coli 0157, Salmonella spp. and Listeria monocytogenes were inoculated into 10 ml of medium No. 17, and cultured at 35°C for 18 h. Each culture solution was taken to a 1 ml-tube, respectively, centrifuged at 15,000 r.p.m for 5 min to collect bacteria. 500 µl of lytic agent (4 M guanidine isothiocyanate solution supplemented with 1 to 2% of Tween 20) was added to the collected bacteria to dissolve collected substances. The resultant solution was heated at 100°C for 10 min, and ice cooled for 5 min. By observing this solution with a light microscope, it was confirmed that pathogenic Escherichia coli 0157 and Salmonella spp. were lysed. The lysis level of Listeria monocytogenes was found lower compared to the lysis level found using the DNA extraction method of Example 3. In addition, lysis of Listeria monocytogenes lysate was performed by adding the following: 1) a solution to which a mixed solution of 10 µl of 20 mg/ml Achromopeptidase and 190 µl of TE buffer was added to the collected bacteria, and treated at 37°C for 1 h; 2) a solution to which a mixed solution of 10 µl of 20 mg/ml of Lysozyme and 190 µl of TE buffer was added to the collected bacteria, and treated at 37°C for 1 h; 3) a solution to which a mixed solution of 10 µl of Enterolysine and 190 µl of TE buffer was added to the collected bacteria, and treated at 37°C for 1 h; 4) a solution to which 300 µl of lysing agent (4 M guanidine isothiocyanate solution supplemented with 1 to 2% of Tween 20) was added to the above 1) solution and mixed; 5) a solution to which 300 µl of lysing agent (4 M guanidine isothiocyanate solution supplemented with 1 to 2% of Tween 20) was added to the above 2) solution and mixed; 6) a solution to which 300 µl of lysing agent (4 M guanidine isothiocyanate solution supplemented with 1 to 2% of Tween 20) was added to the above 3) solution and mixed; 7) a solution to which a mixed solution of 1 µl of 20 mg/ml proteinaze K1 and 200 µl of TE buffer was added to the collected bacteria, and treated at 37°C for 1 h; 8) a solution to which 300 µl of lysing agent (4 M guanidine isothiocyanate solution supplemented with 1 to 2% of Tween 20) was added to the above 7) solution and mixed; 9) a solution to which 500 µl of lysing agent (4 M guanidine isothiocyanate solution supplemented with 1 to 2% of Tween 20) was added to the collected bacteria and mixed. The resultants were observed with a light microscope. In all these conditions, the lysis level of Listeria monocytogenes was lower than the lysis level observed when using the DNA extraction method of Example 3.

### Example 5

### (Determination of PCR reaction condition)

PCR reaction was performed by using the DNA extraction solution obtained in Example 3. For PCR, 1 set of primers specific to target microorganisms to be detected was respectively used from the following primers.
SEQ ID NO: 1: GGC GGA TTA GAC TTC GGC TA
SEQ ID NO: 2: CGT TTT GGC ACT ATT TGC CC
SEQ ID NO: 3: GGG AGT CCA GGT TGA CGG AAA ATT T
SEQ ID NO: 4: GTC ACG GAA GAA GAG AAA TCC GTA CG
SEQ ID NO: 5: CGG AGG TTC CGC AAA AGA TG
SEQ ID NO: 6: CCT CCA GAG TGA TCG ATG TT
SEQ ID NO: 7: ATC ATT GAC GAT TGT AGC ACC
SEQ ID NO: 8: ACA TGA GGA GCA TTA ACT TCG
SEQ ID NO: 9: GGG TCG TTC TAC ATT GAC AG
SEQ ID NO: 10: TTC CCT TTC CAG TAC GCT TC
SEQ ID NO: 11: GTA TTT GGA GAC ATG GGA GC
SEQ ID NO: 12: ACT AAT GAC ACG ATT CGT TCC
SEQ ID NO: 13: CGG ACA GTA GTT ATA CCA C
SEQ ID NO: 14: CTG CTG TCA CAG TGA CAA A
SEQ ID NO: 15: AGC TTT GGT CGT AAA ATA AGG
SEQ ID NO: 16: GAT GCC CAAAGC AGA GAG AT
SEQ ID NO: 17: CAA ACT GCT AAC ACA GCT ACT
SEQ ID NO: 18: GCA CTT GAA TTG CTG TTA TTG
SEQ ID NO: 19: ACC AAT GGG ATC CAC AAG A
SEQ ID NO: 20: GAG CTG AGC TAT GTG CGA T

The PCR solution was made to be a total of 50 µl by adding 5 µl of 10 × Buffer, 4 µl of dNTP solution, 0.5 µl of UNG, 0.25 µl of AmpliTaq Gold, 10 µl of MgCl₂ (all from Applied Biosystems Japan) primers and 2 µl solution of DNA extraction to distilled water. The reaction condition was as follows: maintaining for 2 min at 50°C, allowing to react at 95°C for 10 min, repeating 40 times the cycle of 95°C/20 sec, 60°C/30 sec, 72°C/30 sec, maintaining for 7 min at 72°C and storing at 4°C. PCR products were confirmed by 2.5% agarose gel electrophoresis. When combining DNA consisting of base sequences shown by SEQ ID NOs: 1 to 6, the most ideal primer mixing ratio was: 80 nM each of pathogenic Escherichia coli 0157 primer (SEQ ID NOs: 1, 2); 120 nM each of Salmonella spp. primer (SEQ ID NOs: 3,4); 100 nM each of Listeria monocytogenes primer (SEQ ID NOs: 5, 6). A mixing ratio with low concentration, i.e. 20 nM each of pathogenic Escherichia coli 0157 primers; 30 nM each of Salmonella spp. primers; 25 nM each of Listeria monocytogenes primers was also investigated, which leads to a visual detection by agarose electrophoresis that was somewhat difficult though possible. Further, when DNAs consisting of base sequences shown by SEQ ID Nos: 5 to 10 was used, the most ideal primer mixing ratio were: 240 nM each of pathogenic Escherichia coli 0157 primers (SEQ ID NOs: 7, 8); 60 nM each of Salmonella spp. primers (SEQ ID NOs: 9, 10); 60 nM each of Listeria monocytogenes primer (SEQ ID NOs: 5, 6). Furthermore, it was found that pathogenic Escherichia coli 0157 primers (SEQ ID NOs: 11, 12; or SEQ ID NOs: 13, 14), Salmonella spp. primers (SEQ ID NOs: 15, 16) and Listeria monocytogenes primers (SEQ ID NOs: 17, 18; SEQ ID NOs: 19, 20) could be used by adjusting the mixing ratio, respectively.

Moreover, in the combination of SEQ ID NOs: 5 to 10, it was found that it could be detected by using a probe in which SEQ ID Nos: 21 to 23 specific to the inner sequences are labeled with a fluorescent dye, by detection method such as quantitative PCR or hybridization.
SEQ ID NO: 21 : CGG ATG ATT TGT GGC ACG AGA AA
SEQ ID NO: 22 : TCT GGC ATT ATC GAT CAG TAC CAG CC
SEQ ID NO: 23 : AGT TCAAAT CAT CGA CGG CAA CCT CGG A

### Example 6

### (Confirmation of reaction specificity)

4 strains of pathogenic Escherichia coli 0157, 4 strains of Salmonella spp., 10 strains of Listeria monocytogenes, 4 strains of Escherichia coli other than pathogenic Escherichia coli O 157, and 4 strains of Listeria other than Listeria monocytogenes, shown in Table 2 were used to confirm the specificity of the method. Each bacterium was cultured at 35°C for 18 h with Trypto-soya broth (Nissui Pharmaceutical). Using method 1, among the DNA extractions conditions as defined in Example 3, the extraction method was performed using Achromopeptidase and lysozyme as lytic enzyme. PCR reactions were performed as in Example 5, using the combination of SEQ ID NOs: 1 to 6. Using method 2, among the DNA extractions conditions as defined in Example 3, the extraction method was performed using Enterolysine and lysozyme as lytic enzyme. PCR reactions were performed as in Example 5, using the combination of SEQ ID NOs: 5 to 10. The results of PCR reactions were analyzed on 2.5% agarose gel electrophoresis: bands were detected at expected places for the electrophoresis image of pathogenic Escherichia coli 0157, Salmonella spp. and Listeria monocytogenes, which demonstrates the presence of these bacteria. Meanwhile, for Escherichia coli other than pathogenic Escherichia coli 0157, and Listeria other than Listeria monocytogenes, no bands were detected, which indicated that these bacteria were not present and which confirmed that the method was specific. The results are shown in Table 2.

**[Table 2]**

| | Bacteria | Results | |
|---|---|---|---|
| | | Method 1 | Method 2 |
| 1-1 | Escherichia coli O157:H7 | positive | positive |
| 1-2 | Escherichia coli O157:H7 | positive | positive |
| 1-3 | Escherichia coli O157:H7 | positive | positive |
| 1-4 | Escherichia coli O157:H7 | positive | positive |
| 2-1 | Salmonella Typhimurium | positive | positive |
| 2-2 | Salmonella Enteritidis | positive | positive |
| 2-3 | Salmonella Enteritidis | positive | positive |
| 2-4 | Salmonella sp. | positive | positive |
| 3-1 | Listeria monocytogenes | positive | positive |
| 3-2 | Listeria monocytogenes | positive | positive |
| 3-3 | Listeria monocytogenes | positive | positive |
| 3-4 | Listeria monocytogenes | positive | positive |
| 3-5 | Listeria monocytogenes | positive | positive |
| 3-6 | Listeria monocytogenes | positive | positive |
| 3-7 | Listeria monocytogenes | positive | positive |
| 3-8 | Listeria monocytogenes | positive | positive |
| 3-9 | Listeria monocytogenes | positive | positive |
| 3-10 | Listeria monocytogenes | positive | positive |
| 4-1 | Listeria welshimeri | negative | negative |
| 4-2 | Listeria innocua | negative | negative |
| 4-3 | Listeria ivanovii | negative | negative |
| 4-4 | Listeria seeligeri | negative | negative |
| 5-1 | Escherichia coli 0152 | negative | negative |
| 5-2 | Escherichia coli | negative | negative |
| 5-3 | Escherichia coli | negative | negative |
| 5-4 | Escherichia coli | negative | negative |

### Example 7

(confirmation of detection limit in a meat component-mixed strain) Pathogenic Escherichia coli 0157, Salmonella spp. and Listeria monocytogenes of Example 1 were used. Each bacterium was inoculated in 10 ml of the above-mentioned medium No. 17, cultured at 35°C for 18 h, and bacteria culture solution was obtained. In parallel, 225ml of the medium no. 17 was added to 25 g of minced chicken leg, which was ground with a stomacher for 30 sec and cultured at 35°C for 18 h. General viable cell counts at that time were 3.1 × 10⁸ CFU/ml. Culture solution of minced chicken leg was poured by aliquots of 9 ml. Subsequently, 1 ml of 10-fold serial diluted solution of each bacteria culture solution was added to prepare meat sample solution for each diluted series of each bacteria culture solution. Large food scraps were removed by passing each sample solution through a 5 µl-filter. DNA extraction method as defined in Example 3 was performed using the following conditions: the extraction method using Achromopetidase and lysozyme as lytic enzyme. PCR reactions were carried out as defined in Example 5 using the combination of SEQ ID NOs: 1 to 6. Furthermore, DNA extractions were also performed following method as defined in Example 3, using Enterolycine and lysozyme. PCR reactions were carried out as defined in Example 5, using the combination of SEQ ID NOs: 5 to 10. Each of the PCR product was confirmed by 2.5% agarose gel electrophoresis, and the detection limit of pathogenic Escherichia coli 0157, Salmonella spp. and Listeria monocytogenes in meat sample solution was confirmed to be 10³ CFU/ml, respectively. The electrophoresis figure showing the result of method 1 is shown in Fig. 2 as representative.

### Example 8

### (Detection of pathogenic bacteria from inoculated food)

Pathogenic Escherichia coli O 157, Salmonella spp. and Listeria monocytogenes of Example 1 were used. Each bacterium was inoculated till they reach 10² CFU/25 g, 10 CFU/25g, 1 CFU/25 g, 10⁻¹ CFU/25 g to minced pork meat respectively. In parallel, 225 ml of the medium no. 17 was added to 25 g of inoculated minced pork meat, which was ground with a stomacher for 30 sec and cultured at 35°C for 24 h. After removing large food scraps by passing each sample solution through a 5 µl-filter. DNA extractions were performed as in Example 3 using method 1 with using Achromopetidase and lysozyme as lytic enzyme. The PCR reactions were performed as in Example 5:using the combination of SEQ ID NOs: 1 to 6. DNA extractions were also performed following method 2 as in Example 3 using Enterolycine and lysozyme. The PCR reactions were performed as in Example 5 using the combination of SEQ ID NOs: 5 to 10. The results given by each method were analyzed by 2.5% agarose gel electrophoresis. The results obtained using method 1 are shown in Fig. 3. As a result, it was confirmed that all of the bacteria could be detected with any of the two methods, as long as the bacteria to be detected are present in an amount of 1 CFU/25 g. Harmful pathogenic bacteria such as pathogenic Escherichia coli 0157, Salmonella spp. and Listeria monocytogenes, are set up to be "negative" in foods (not included in 25 g), and a comparable or even superior accuracy compared to that of official methods is required for the detection. The present multiplex detection method was confirmed to have comparable or even superior accuracy compared to official methods.

### Example 9

### (Comparable test of official methods with the multiplex detection method; detection of pathogenic bacteria from foods on market)

20 samples including chicken meat and chicken liver were purchased at supermarkets. Inspection of pathogenic Escherichia coli, Salmonella spp. and Listeria monocytogenes were performed by the multiple detection method and the results were compared with those of official methods. The official method was performed as follows.

For pathogenic Escherichia coli 0157, 225 ml of novobiocin mEC broth (Kyokuto Pharmaceutical Industrial) was added to 25 g of sample, which was ground with a stomacher for 30 sec, cultured at 42°C for 18 hours, streaked in CHROMagar 0157 medium (Kanto Chemical) and MacConkey Sorbitol Agar medium (Nissui Pharmaceutical), and cultured at 35°C for 18 to 24 h. Those who showed lilac in CHROMagar 0157 medium, and translucent pink in MacConkey Sorbitol Agar medium were determined to be false-positive pathogenic Escherichia coli 0157, and were streaked in CLIG agar medium (Kyokuto Pharmaceutical Industrial) and cultured at 35°C for 18 to 24 h. Those whose lower layer was yellow and upper layer was pink, and that were not luminescent to ultraviolet radiation were determined to be false-positive pathogenic Escherichia coli 0157. They were subjected to indole reaction, and for those being positive (red), agglutination reaction was performed. Agglutination reaction was performed by using the Escherichia coli 0157 detection kit "UNI" (Oxoid). Colonies that were doubtful by agglutination reaction were streaked in CHROMagar 0157 medium, MacConkey Sorbitol agar medium, TSI agar medium (Nissui Pharmaceutical) and LIM medium (Nissui Pharmaceutical), and were cultured at 35°C for 24 hours. Those who showed lilac in CHROMagar 0157 medium, translucent pink in MacConkey Sorbitol agar medium, yellow in TSI agar medium and showing no change in LIM medium, were confirmed to be pathogenic Escherichia coli 0157 by PCR reaction.

For Salmonella spp., 225 g of EEM broth (Nissui Pharmaceutical) was added to 25 g of sample, which was ground with a stomacher for 30 sec. The resultant was cultured at 35°C for 18 h, and 1 ml thereof was added to 10 ml of Selenite-Cystine base medium (Nissui Pharmaceutical), and cultured at 43°C for 15 to 18 h. For those wherein the whole or the precipitation showed red, 1-loopful was streaked in MLCB agar medium (Nissui Pharmaceutical) and cultured at 35°C for 24 h. Those who produced black colonies were determined to be false-positive Salmonella spp., and as a confirmation test, these were streaked in TSI agar medium and LIM agar medium. After culturing at 35°C for 24 to 48 h, those who showed red slope, black in upper layer in TSI medium, and showed no change in LIM medium, were determined to be positive Salmonella spp.

For Listeria monocytogenes, 225 ml of UVM Listeria selective enrichment bouillon (Merck) was added to 25 g of sample, which was ground with a stomacher for 30 sec. After culturing at 30°C for 48 h, 1-loopful was streaked in PALCAM Listeria selective agar medium (Merck). Those who were determined to be positive Listeria after culturing at 35° C for 48 h, were streaked in horse blood agar medium (Nissui Pharmaceutical), Standard agar medium (Nissui Pharmaceutical) and cultured at 35°C for 24 to 48 h. Those whose hemolytic was positive, were subjected to oxydase reaction, catalase reaction, gram staining, microscope observation, Api Listeria (Japan Biomerieux) and those who were identified as Listeria monocytogenes were determined to be positive Listeria monocytogenes.

The multiplex detection method was performed as follows. 225 ml of medium No. 17 was added to 25 g of sample, which was ground with a stomacher for 30 sec, and cultured at 35°C for 24 h. Large food scraps were removed by passing the culture solution through a 5 µl-filter. DNA extractions were performed using method 1 as in Example 3, using Achromopetidase and lysozyme as lytic enzyme. The PCR reactions were performed as in Example 5 using the combination of SEQ ID NOs: 1 to 6. Further, using method 2 , DNA extractions were performed as in Example 3, using Enterolysine and lysozyme as lytic enzyme. PCR reactions were performed as in Example 5, using the combination of SEQ ID NOs: 5 to 10. Each of the PCR reaction product was confirmed by 2.5% agarose gel electrophoresis. The results are shown in Table 3.

As a result, samples in which pathogenic bacteria were detected were as follows. By the official method: pathogenic Escherichia coli O157: 0 sample; Salmonella spp.: 3 samples; Listeria monocytogenes: 6 samples. By any of the multiplex detection method: pathogenic Escherichia coli O157: 0 sample; Salmonella spp.: 3 samples; Listeria monocytogenes: 8 samples. Those who were positive by the official method were all positive by the multiple detection method. Moreover, as for 2 samples of Listeria monocytogenes who were negative by the official method and positive by the multiple detection method (Table 3; sample No. 5, 15), the culture solution in No. 17 was streaked and cultured in PALCAM Listeria selective agar medium (Merck), and the formed colonies were subjected to an identification test. As a result, they were confirmed to be Listeria monocytogenes. From this result, the present multiplex detection method was confirmed to have more accuracy than the official method.

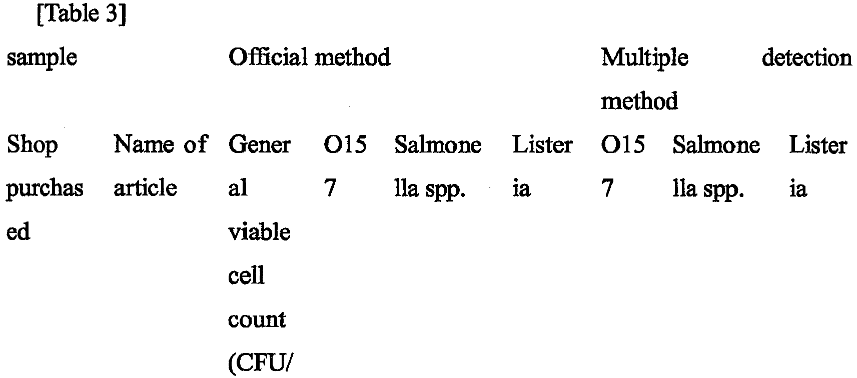

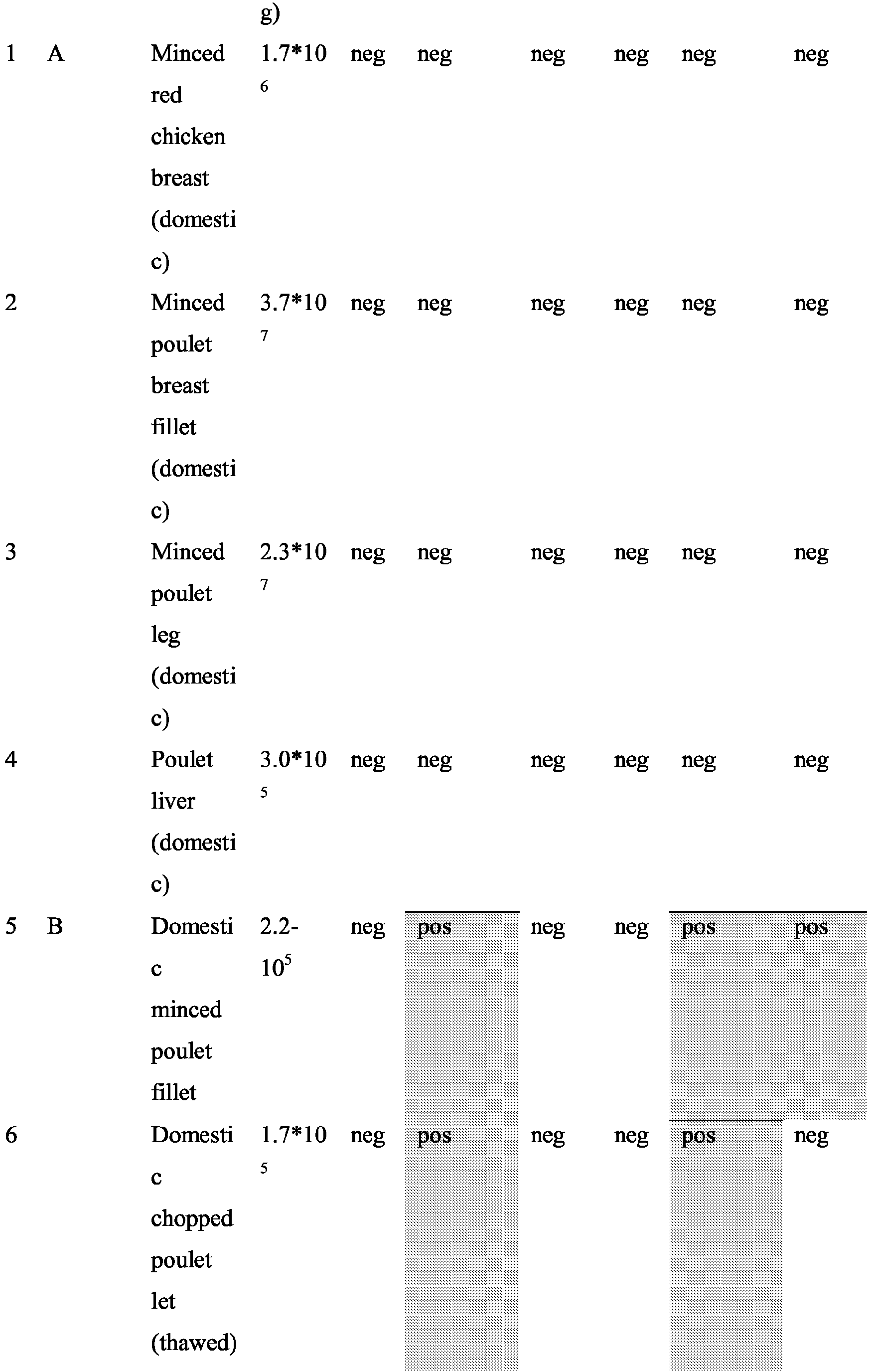

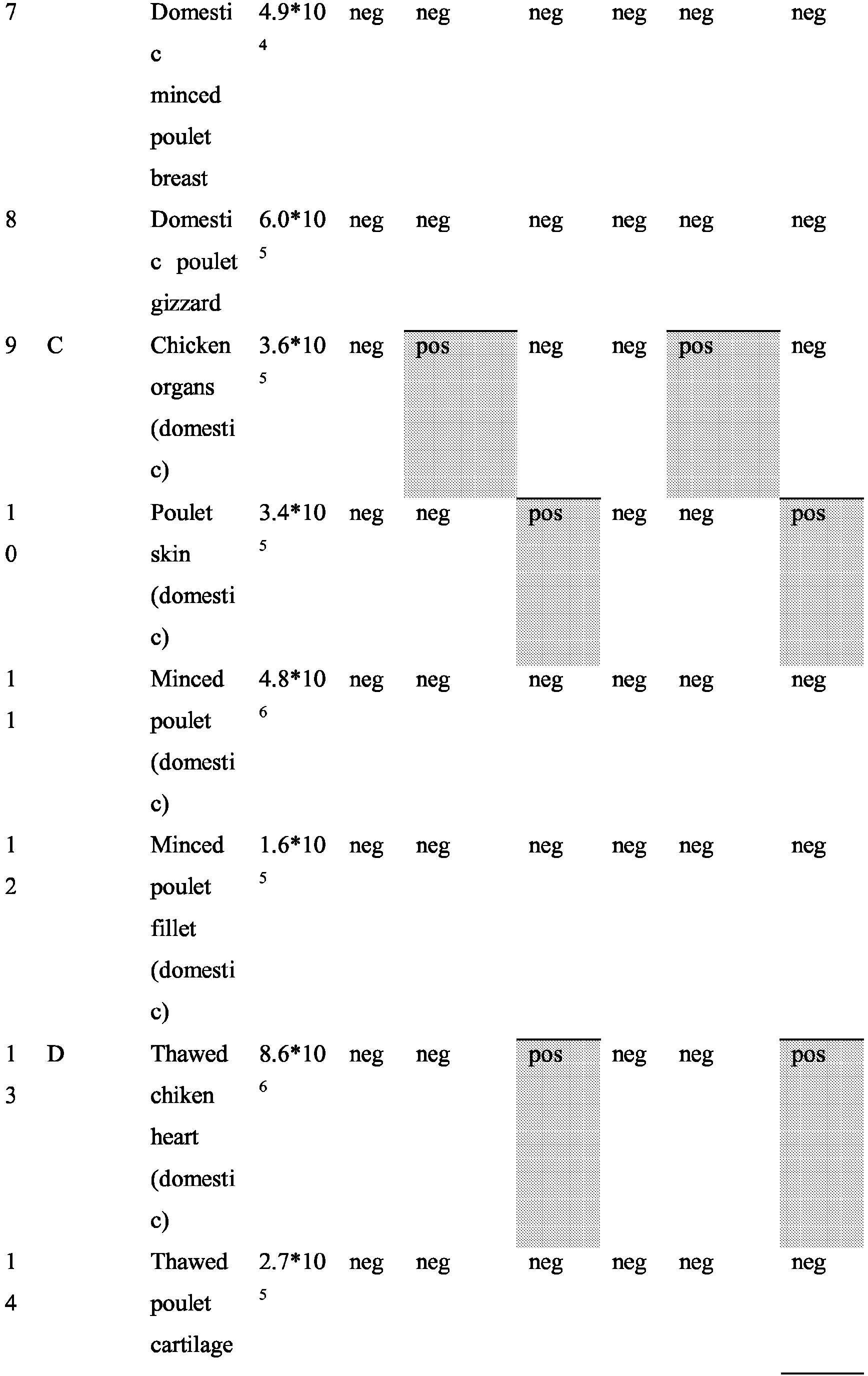

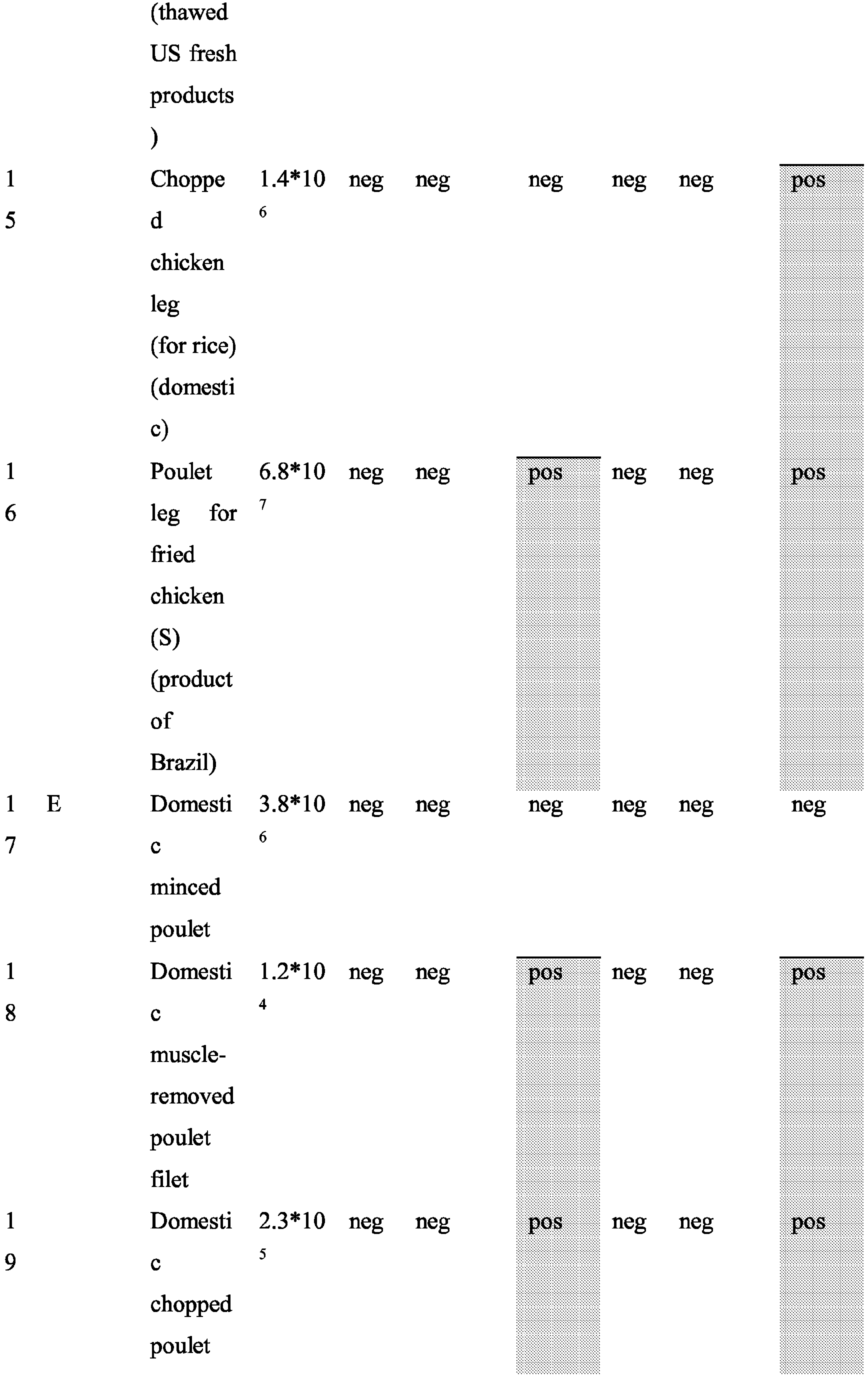

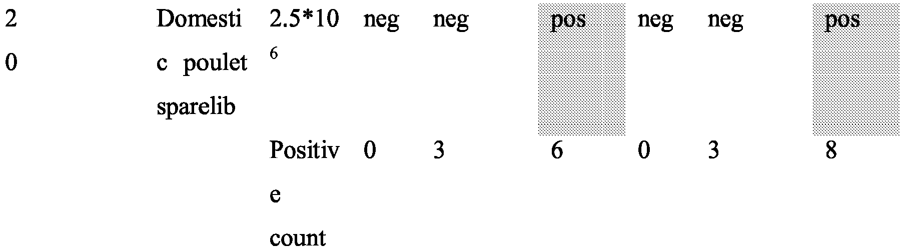

### Brief Description of Drawings

[Fig. 1] It is a figure showing change of the counts of pathogenic Escherichia coli 0157, Salmonella spp., Listeria monocytogenes and general viable cells in the culture at 35°C using Trypto-soya broth(TSB) and Buffered Peptone Water (BPW).
[Fig. 2] It is a figure of the electrophoresis image showing the detection limit by the multiple detection method of each bacterium in the meat sample solution (general viable cell counts: 3.1 × 10⁸ CFU/ml) inoculated with each bacterium of pathogenic Escherichia coli 0157, Salmonella spp. and Listeria monocytogenes.
   M: molecular weight marker
   Lanes 1 to 7 show the pathogenic Escherichia coli-inoculated region (general viable cell counts: 3.1 × 10⁸ CFU/ml), and the inoculated amount of 0157 is as follows:
   1: 1.1 × 10⁷ CFU/ml; 2: 1.1 × 10⁶ CFU/ml; 3: 1.1 × 10⁵ CFU/ml;
   4: 1.1 × 10⁴ CFU/ml; 5: 1.1 × 10³ CFU/ml; 6: 1.1 × 10² CFU/ml; 7: 11 CFU/ml

   Lanes 8 to 14 show the Salmonella spp.-inoculated region (general viable cell counts: 3.1 × 10⁸ CFU/ml), and the inoculated amount of Salmonella spp. is as follows: 8: 5.0 × 10⁶ CFU/ml; 9: 5.0 × 10⁵ CFU/ml; 10: 5.0 × 10⁴ CFU/ml; 11: 5.0 × 10³ CFU/ml; 12: 5.0 × 10² CFU/ml; 13: 50 CFU/ml;
   14: 5 CFU/ml
   Lanes 15 to 21 show the Listeria monocytogenes-inoculated region (general viable cell counts: 3.1 × 10⁸ CFU/ml), and the inoculated amount of Listeria monocytogenes is as follows:
   15: 1.1 × 10⁷ CFU/ml; 16: 1.1 × 10⁶ CFU/ml;
   17: 1.1 × 10⁵ CFU/ml; 18: 1.1 × 10⁴ CFU/ml;
   19: 1.1 × 10³ CFU/ml; 20: 1.1 × 10² CFU/ml; 21: 11 CFU/ml
[Fig. 3] It is a figure of the electrophoresis image showing the result of detection of each bacterium by the multiple detection method, using minced pork meat inoculated with pathogenic Escherichia coli 0157, Salmonella spp. and Listeria monocytogenes.
   M: molecular weight marker
   Lanes 1 to 4 show the Listeria monocytogenes-inoculated region, and the inoculated amount of Listeria monocytogenes is as follows:
   1: 16 CFU/25g; 2: 1.6 CFU/25g; 3: 0.16CFU/25g;
   4: 0.02 CFU/25g

   Lanes 5 to 8 show the Salmonella spp.-inoculated region, and the inoculated amount of Salmonella spp. is as follows:
   5: 110 CFU/25g; 6: 11 CFU/25g; 7: 1.1 CFU/25g;
   8: 0.11 CFU/25g

   Lanes 9 to 12 show the pathogenic Escherichia coli 0157-inoculated region, and the inoculated amount of 0157 is as follows:
   9: 850 CFU/25g; 10: 8.5 CFU/25g; 11: 8.5 CFU/25g;
   12: 0.85 CFU/25g

## Claims

1. A method for multiplex microorganism detection which is a method for detecting microorganisms including Listeria monocytogenes, pathogenic Escherichia coli 0157 and Salmonella spp. in foods, by amplifying a plurality of target genes in a single PCR reaction tube, comprising the following steps:
(A) a step for culturing microorganisms under such culture conditions as allowing the proliferation of each microorganism of pathogenic Escherichia coli 0157, Salmonella spp., and Listeria monocytogenes for 18 h or more in a medium with a concentration of phosphate-buffer solution of 50 mM or more or having a buffer ability, which is similar to the one of a medium having a concentration of phosphate-buffer solution of 50 mM or more;
(B) a step for extracting DNA of the target microorganisms to be detected, by treating with at least a lytic enzyme and /or a bacteriocin having a lytic activity, further treating with a surfactant and a protein-denaturing agent; and
(C) a step for carrying out Multiplex PCR after having mixed a combination of
(i) a DNA selected from the group consisting of primer sets for pathogenic Escherichia coli O157: SEQ ID Nos: 1 and 2, SEQ ID Nos: 7 and 8, SEQ ID Nos: 11 and 12, and SEQ ID Nos: 13 and 14,
(ii) a DNA selected from the group consisting of primer sets for Salmonella spp.: SEQ ID Nos: 3 and 4, SEQ ID Nos: 9 and 10, and SEQ ID Nos: 15 and 16, and
(iii)a DNA selected from the group consisting of primer sets for Listeria monocytogenes: SEQ ID Nos: 5 and 6, SEQ ID Nos: 17 and 18, and SEQ ID Nos: 19 and 20,
as primer sets.

2. The method according to claim 1, wherein the microorganisms are cultured in such culture conditions allowing the pH to reach a value of 5.1 or higher after the completion of the culture.

3. The method according to claim 1 or 2, wherein the step (B) is performed by treating with a lytic enzyme and/or a bacteriocin having a lytic activity, further treating with a surfactant and a protein-denaturing agent, removing insoluble fractions by centrifugation, and depositing DNA by alcohol precipitation.

4. The method according to any one of claims 1 to 3, wherein the lytic enzyme is Achromopeptidase and/or lysozyme.

5. The method according to any one of claims 1 to 4, wherein bacteriocin having lytic activity is Enterolysine.

6. The method according to any one of claims 1 to 5, wherein the surfactant is ethyleneoxide condensate of sorbitan monolaurate.

7. The method according to any one of claims 1 to 6, wherein the protein-denaturing agent is Guanidine isothiocyanate.

8. The method according to any one of claims 1 to 7, wherein the food is edible meat or processed meat product.

## Patentansprüche

1. Verfahren zum Nachweis von Mikroorganismen mittels Multiplex-Analyse, welches ein Verfahren zum Nachweis von Mikroorganismen, umfassend Listeria monocytogenes, pathogene Escherichia coli 0157 und Salmonella spp. in Nahrung ist, durch Amplifizieren einer Vielzahl von Zielgenen in einem einzigen PCR Reaktionsgefäß, umfassend die folgende Schritte:
(A) ein Schritt zur Kultivierung von Mikroorganismen unter solchen Kultivierungsbedingungen, die die Proliferation von jedem Mikroorganismus von pathogenem Escherichia coli 0157, Salmonella spp. und Listeria monocytogenes für 18 Stunden oder länger in einem Medium mit einer Konzentration an Phosphat-Puffer Lösung von 50 mM oder mehr oder mit einer Pufferfähigkeit, die ähnlich der Pufferfähigkeit eines Mediums mit einer Konzentration an Phosphat-Puffer Lösung von 50 mM oder mehr ist, zulassen;
(B) ein Schritt zum Extrahieren von DNA der nachzuweisenden Zielmikroorganismen durch Behandeln mit mindestens einem lytischen Enzym und/oder einem Bakteriozin mit lytischer Aktivität, und weiter durch Behandeln mit einem Tensid und einem Proteindenaturierungsagenten; und
(C) ein Schritt zur Durchführung von Multiplex-PCR nachdem die folgende Kombination als Primer-Sets gemischt wurde:
(i) eine DNA, die ausgewählt ist aus der Gruppe bestehend aus Primer-Sets für pathogene Escherichia coli O157: SEQ ID Nr.: 1 und 2, SEQ ID Nr.: 7 und 8, SEQ ID Nr.: 11 und 12, und SEQ ID Nr.: 13 und 14,
(ii) eine DNA, die ausgewählt ist aus der Gruppe bestehend aus Primer-Sets für Salmonella spp.: SEQ ID Nr.: 3 und 4, SEQ ID Nr.: 9 und 10, und SEQ ID Nr.: 15 und 16, und
(iii) eine DNA, die ausgewählt ist aus der Gruppe bestehend aus Primer-Sets für Listeria monocytogenes: SEQ ID Nr.: 5 und 6, SEQ ID Nr.: 17 und 18, und SEQ ID Nr.: 19 und 20.

2. Verfahren nach Anspruch 1, bei dem die Mikroorganismen unter solchen Kultivierungsbedingungen kultiviert werden, die das Erreichen eines pH Werts von 5,1 oder höher nach Beendigung der Kultur ermöglichen.

3. Verfahren nach Anspruch 1 oder 2, bei dem Schritt (B) durch Behandeln mit einem lytischen Enzym und/oder einem Bakteriozin mit lytischer Aktivität, weiter durch Behandeln mit einem Tensid und einem Proteindenaturierungsagenten, Entfernen unlöslicher Fraktionen durch Zentrifugation, und Fällen von DNA durch Alkohol Präzipitation durchgeführt wird.

4. Verfahren nach einem der Ansprüche 1 bis 3, bei dem das lytische Enzym Achromopeptidase und/oder Lysozym ist.

5. Verfahren nach einem der Ansprüche 1 bis 4, bei dem das Bakteriozin mit lytischer Aktivität Enterolysin ist.

6. Verfahren nach einem der Ansprüche 1 bis 5, bei dem das Tensid Ethylenoxid Kondensat von Sorbitanmonolaurat ist.

7. Verfahren nach einem der Ansprüche 1 bis 6, bei dem der Proteindenaturierungsagent Guanidinisothiocyanat ist.

8. Verfahren nach einem der Ansprüche 1 bis 7, bei dem die Nahrung essbares Fleisch oder ein verarbeitetes Fleischprodukt ist.

## Revendications

1. Procédé de détection multiplex de microorganismes permettant la détection de microorganismes tels que la Listeria monocytogène, l'Escherichia coli 0157 pathogène et la Salmonella spp. dans des aliments, en amplifiant plusieurs gènes cibles dans un seul tube de réaction PCR, comprenant les étapes suivantes :
(A) une étape de culture de microorganismes dans des conditions de culture permettant la prolifération de chaque microorganisme d'Escherichia coli 0157 pathogène, de Salmonella spp. et de Listeria monocytogène pendant une durée supérieure ou égale à 18 h dans un milieu ayant une concentration de solution tampon phosphate supérieure ou égale à 50 mM ou présentant une capacité tampon semblable à celle d'un milieu ayant une concentration de solution tampon phosphate supérieure ou égale à 50 mM ;
(B)une étape d'extraction d'ADN des microorganismes cibles à détecter, par un traitement par au moins un enzyme lytique et/ou une bactériocine ayant une activité lytique, suivi d'un traitement par un agent tensioactif et un agent de dénaturation de protéine ; et
(C)une étape de réalisation d'une PCR multiplex après avoir mélangé une combinaison de
(i) un ADN choisi dans le groupe comprenant les ensembles d'amorces pour l'Escherichia coli 0157 pathogène : SEQ ID n°1 et 2, SEQ ID n°7 et 8, SEQ ID n°11 et 12 et SEQ ID n°13 et 14,
(ii) un ADN choisi dans le groupe comprenant les ensembles d'amorces pour la Salmonella spp. : SEQ ID n°3 et 4, SEQ ID n°9 et 10, SEQ ID n°15 et 16 et
(iii) un ADN choisi dans le groupe comprenant les ensembles d'amorces pour la Listeria monocytogène : SEQ ID n°5 et 6, SEQ ID n° 17 et 18 et SEQ ID n° 19 et 20,
en tant qu'ensembles d'amorces.

2. Procédé selon la revendication 1, dans lequel les microorganismes sont cultivés dans des conditions de culture permettant au pH d'atteindre une valeur supérieure ou égale à 5,1 une fois la culture menée à bien.

3. Procédé selon la revendication 1 ou 2, dans lequel l'étape (B) est réalisée par traitement par un enzyme lytique et/ou une bactériocine ayant une activité lytique, suivi d'un traitement par un agent tensioactif et un agent de dénaturation de protéine, d'un retrait des fractions insolubles par centrifugation et d'un dépôt d'ADN par précipitation alcoolique.

4. Procédé selon l'une quelconque des revendications 1 à 3, dans lequel l'enzyme lytique est une achromopeptidase et/ou un lysozyme.

5. Procédé selon l'une quelconque des revendications 1 à 4, dans lequel la bactériocine ayant une activité lytique est l'entérolysine.

6. Procédé selon l'une quelconque des revendications 1 à 5, dans lequel l'agent tensioactif est un condensé d'oxyde d'éthylène de monolaurate de sorbitane.

7. Procédé selon l'une quelconque des revendications 1 à 6, dans lequel l'agent de dénaturation de protéines est l'isothiocyanate de guanidine.

8. Procédé selon l'une quelconque des revendications 1 à 7, dans lequel les aliments sont de la viande comestible ou un produit camé transformé.
